# EUROPEAN PATENT SPECIFICATION

(11) **EP 0 617 935 B1**
(45) Date of publication and mention of the grant of the patent: **03.03.1999**
(21) Application number: 94302231.9
(22) Date of filing: 29.03.1994
(51) Int. Cl.: A61F 2/46

(54) **Apparatus for removal of osteal prostheses**
Gerät zur Entfernung von Knochenprothesen
Appareil pour retirer une prothèse osseuse

(43) Date of publication of application: 05.10.1994
(60) Divisional application: 97112886.3
(73) Proprietor: YOUNG, Michael John Radley, Ashburton, South Devon TQ13 7JX (GB); BRADNOCK, Brian Robert Denis Peter, Hemel Hempstead, Hertfordshire HP3 0PD (GB)
(72) Inventor: YOUNG, Michael John Radley, Ashburton, South Devon TQ13 7JX (GB); BRADNOCK, Brian Robert Denis Peter, Hemel Hempstead, Hertfordshire HP3 0PD (GB)
(74) Representative: Gregory, Timothy Mark

(56) References cited:
- DE-A- 4 041 063

## Description

The invention relates to ultrasonic and means for removing an osteal prosthesis from cemented installation in a living bone, as in the course of revision arthroplasty.

It is known from U.S. **Patents 4,248,232 and 5,151,099** that ultrasound may be used to facilitate removal of bone cement (PMMA) during revision arthroplasty. Local heating, by preferential absorption of ultrasound energy, raises the temperature of a small volume of the cement above the glass-transition temperature, thus allowing the cement to flow and to be manipulated into a shape and form which may be readily removed from the revision site.

It has also been claimed (**Hood, et al., U.S. Patent 5,045,054**) that the application of ultrasound directly to the prosthesis can break the bond between the prosthesis and surrounding cement or, in the case of uncemented prostheses, between the prosthesis and in-grown cancellous bone. The direction of applied ultrasonic energy is in line with the central axis of an implanted prosthetic devise, such as the axis of stem support for the ball of a hip-joint replacement. But the in-line application of force, as in the context of the **Hood, et al.** system, is to require the patient to oppose the force, with inevitable trauma for the patient.

It is an object of the invention to provide improved means for dislodging an installed osteal prosthesis with a minimum of trauma for the patient who is faced with the need of revision arthroplasty.

A specific object is to meet the above object for the case of revision arthroplasty of a hip-joint prosthesis that has been implanted and cemented in a femur.

It is a general object to meet the above objects with an apparatus of reliably high performance without a daunting level of operating complexity, which method and apparatus are applicable to the full variety of currently available prostheses.

The invention in its preferred embodiment allows an ultrasonically driven technique to be carried out wherein an annular body of relatively great mass is solidly chucked around the exposed proximal end of a hip-joint prosthesis which has been cemented in the medullary canal of a femur. In most cases, the exposed proximal end is a sphere or ball at the projecting end of a stem, all integrally formed with the implanted remainder of the prosthetic device. The annular body is excited into ultrasonic radial-mode oscillation by a driver having a directional axis of mechanical oscillation, wherein the said axis is preferably radially inward through body material, toward the body axis, and preferably for substantial alignment with the centre of the exposed ball or head end of the prosthesis to be removed, as for prosthetic replacement in the patient. In response to such excitation, the annular body reacts with radial-mode resonant oscillation, involving circumferentially continuous application of radially modulated squeezing transfer of ultrasonic energy into the prosthetic at the region of chucked engagement; and, in turn, the prosthetic responds to the radial-mode resonance by such plastic-deformation of its cemented and/or bony-ingrowth attachment to the patient's limb, as to locally generate enough heat to melt cementing plastic at interface with the prosthetic, or to mechanically sever bony ingrowth at interface with the prosthetic. The ultrasonically driven body annulus is of such design as to selectively accommodate a variety or sizes of prosthetic-head chucked engagement. The body annulus will also accommodate a special tool which may be selected from one of a group of chucks, each of which is configured to coupling to the buried end of a broken fragment of a prosthetic shaft, with an ability to transfer sufficient ultrasonic energy through the tool and into the broken and buried prosthetic fragment, for clean and efficient dislodging and extraction of the buried fragment.

According to one aspect of the invention, there is provided a radial-mode resonant device characterised in that it comprises an annular body having a central axial bore and an ultrasonic driver having a central axis of mechanically resonant oscillation directed generally radially inwardly with respect to the central axis of said body.

Preferably, there is provided an ultrasonically driven tool comprising a collet means in combination with a radial-mode resonant device as described above, characterised in that the collet means comprises resiliently deformable means to define an engagement zone whereby energy from said resonant device may be delivered to said prosthesis.

Embodiments of the present invention will now be more particularly described by way of example and with reference to the accompanying drawings, in which:
**Fig. 1** is a view in side elevation, partly broken-away and in section, to show elements of a prosthetic-removal device of the invention;
**Fig. 2** is a view similar to **Fig. 1** to show a modification;
**Fig. 3** is an exploded view of separable parts, as in **Fig. 2**;
**Fig. 4** is a plan view of the device of **Fig. 3;**
**Fig. 5** is a view similar to **Fig. 1** to show another modification;
**Fig. 6** is a view similar to **Fig. 1** to show a further modification;
**Fig. 7** is a view in longitudinal section of a tool element usable in conjunction with any of the devices of **Figs. 1, 2, 3, 5 or 6,** for removal of a distal broken end of prosthetic device;
**Fig. 8** is a sectional view to show use of the tool of **Fig. 7** in the device of **Fig. 6**;
**Fig. 9** is an electrical block diagram to show excitation and control circuitry for use in any of the disclosed prosthetic-removal embodiments of the invention; and
**Fig. 10** is a graph to illustrate a frequency band characteristic of ultrasonic operation, using circuity of **Fig. 9**.

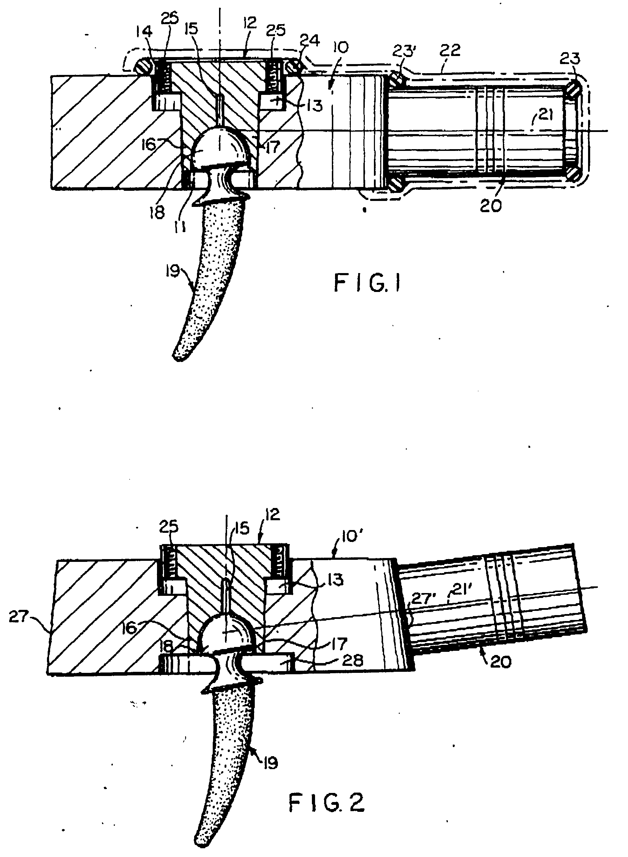

Referring initially to **Fig. 1**, the invention is shown in application to a radial-mode oscillator comprising an annular body 10 of relatively great mass, suitably of stainless steel. body 10 is of generally right-cylindrical configuration and has a central bore 11 which is slightly convergent in the downward direction, for the purpose of coaction with a chuck or collet member 12. At its upper end, bore 11 expands via a counterbore 13 which will accommodate the radially flanged head end 14 of member 12.

The chuck or collect member 12 has a reduced distally extending lower end which is longitudinally split, as at slit 15, to define plural distal fingers, here shown as the two fingers 16, 17, in view of the single slit 15.

In the case of **Fig. 1**, the exposed spherical head or ball 18 of an installed hip prosthesis 19 is securely engaged by concave spherical formations within the distal or finger end of member 12, i.e., within confronting internal concave spherical surfaces of the two fingers 16, 17, it being understood that the concavities of fingers 16, 17 cooperatively define a hemispherical socket against which a ball head 18 intimately nests. The external surfaces of fingers 16, 17 preferably conform to convergence of the body bore 11 such that, once the ball head 18 has been inserted into the unstressed socket defined by the internal concave spherical surfaces of fingers 16, 17, the collet or chuck member 12 may be driven downward to securely and circumferentially continuously engage and grip the ball head 18. The drive to establish such a grip can be obtained by hammer blows delivered axially to the flanged upper end 14; in the course of such a drive, fingers 16, 17 are inwardly deflected by wedge action between the convergent concave and convex tapering surfaces, and it will be understood that in a finally chucked position, as in **Fig. 1**, the ball head 18 not only has virtually equatorial grip by the fingers 16, 17, but that this grip is similarly circumferential for axially extending areas above and below the equatorial location. Alternatively, the surgeon may be of the view that hammer blows might result in trauma to the patient, in which case a pair of opposed C-clamps applied to squeeze the flange of member 12 with respect to the lower annular surface of body 10 is indicated, for a reduced likelihood of trauma.

To excite the described body 10 and its securely chucked prosthetic device 19, an electromechanical transducer 20 is shown secured locally to the periphery of body 10. Transducer 20 is suitably of the piezoelectric ceramic variety generally as shown and described (at reference number 1) in **U.S. Patents 5,151,099** and **5,536,266** to which reference is made for greater detail. Transducer 20 is cylindrical and has a central axis 21 of mechanically resonant oscillation, at a frequency in the range 20 kHz to 40 kHz. The transducer is driven as the electrical load of circuitry to be described later in connection with **Fig. 9**, but which will be indicated here to feature a phase-locked loop for automatic High-Q tuning over a range of frequencies. The range of frequency adjustment will be understood to be that which best serves the mechanically resonant properties of body 10 and its well chucked connection to prosthetic 19, and the latter will be further understood to be in its embedded condition, and to have been the subject of an arthroscopic femoral procedure which now requires replacement.

To give an indication of size, a radial-mode resonant body 10 to accommodate a ball head 18 of 0.0254m (1 inch) diameter is suitably of 0.127 to 0.152m (5 to 6 inches) diameter, with an axial thickness of 0.051m (2 inches); and transducer 20 may suitably be of 0.076 to 0.089m (3 to 3.5 inches) overall length and 0.254 to 0.040m (1 to 1.6 inches) diameter.

A housing recommended for the described parts may generally be as shown and described in **US Patent 5,536,266** as long as the housing enables safe handling during ultrasonic operation. The phantom outline 22, with elastomeric O-ring supports at 23, 23', 24 will be understood to be suggestive of such a housing. The housing 22 happens to be removable, cylindrically surrounding the transducer 20 and thus providing a handle that is mechanically insulated from transducer resonance, and with an upper looping ring portion removably centred on and surrounding the flanged upper end of the chuck or collet member 12.

In operation, excitation of transducer 20 induces radial-mode resonance in body 10 and in the chuck and ball-head elements securely bound within body 10. The ball-head is thus induced to track the excursions of this mechanical resonance and to couple them into the volume of the prosthetic 19, with resultant complex mechanical oscillation (featuring multiple nodes/antinodes) at interface between the shank of the prosthetic and such plastic cement or other bond (such as bony ingrowth) that may exist. The result is rapidly, within seconds, to melt cement and to shear bony ingrowth and thus to enable manual retraction of the radial-mode system and the prosthetic 19.

In the course of such retraction, which can be relatively quickly accomplished (in view of the tapered nature of the embedded prosthetic stem), it is optional whether or not the transducer remains excited, because the embedment bond to the patient will have been severed.

A note should be made to the effect that the prosthetic appliance 19 shown for illustration herein is an integrally formed single piece, so that a dislodgement is of the entire appliance. There are, however, other appliance structures in use for the same kind of hip-joint replacement. For example, the ball head 18 may have been a separately manufactured ball having a radial bore for "permanent" Morse-taper fit to an otherwise exposed stud which is an integral part of the prosthetic. In that event, the described ultrasonic radial-mode excitation of the exposed ball of the prosthetic appliance may result in dislodging the ball from its Morse-taper fit, thus exposing the tapered stud portion of an appliance that remains embedded in the patient. For such a situation, it will be understood that a second radial-mode system as described for **Fig. 1** may be at hand and equipped with a chuck or collet having internal concave contours suited for axially extensive and for virtually circumferentially continuous engagement with the otherwise exposed stud end of the prosthetic. Ultrasonic excitation of the chucked stud will then achieve the same desired result of inducing such mechanical action at the embedded interface or interfaces within the body as to enable quick and efficient retraction of the prosthetic.

Once the prosthetic has been removed, personnel aiding the surgeon can address the problem of disengaging the chuck and the chucked prosthetic from the radial-mode body. To this end, angularly spaced plural tapped bores 25 in the flanged end of the chuck may be threaded with bolts (not shown), for axially jacking reference to the flat inner annular end of the counterbore 13. Upon a sufficiently jacked displacement, the chuck action becomes dislodged and the prosthetic and the chuck 12 may be removed from each other and from body 10.

The embodiment of **Fig. 2** represents slight modification from what has been described in **Fig. 1**, and the same reference numbers are reused where possible. The chief difference in **Fig. 2** is that the directional axis 21' of ultrasonic excitation by transducer 20 is not only orientated radially inward, but axis 21' is also inclined downwardly for intersection at or near the spherical center of the concave spherical inner surfaces of fingers 16, 17, thus at or near the spherical center of a ball or ball head 18 chucked thereto. To this end, the annular body 10' for excitation into radial-mode mechanical oscillation has an outer surface 27 which is frusto-conical so that the inclined driving end face of transducer 20 may be mounted to a locally milled flat 27' in the frusto-conical outer surface. Further, **Fig. 2** shows an additional counterbore 28 at the lower end of the central bore of body 10, to provide greater concentration of ultrasonic energy from body 10' structure to an exposed ball head and an associated bone structure (not shown in **Fig. 2**).

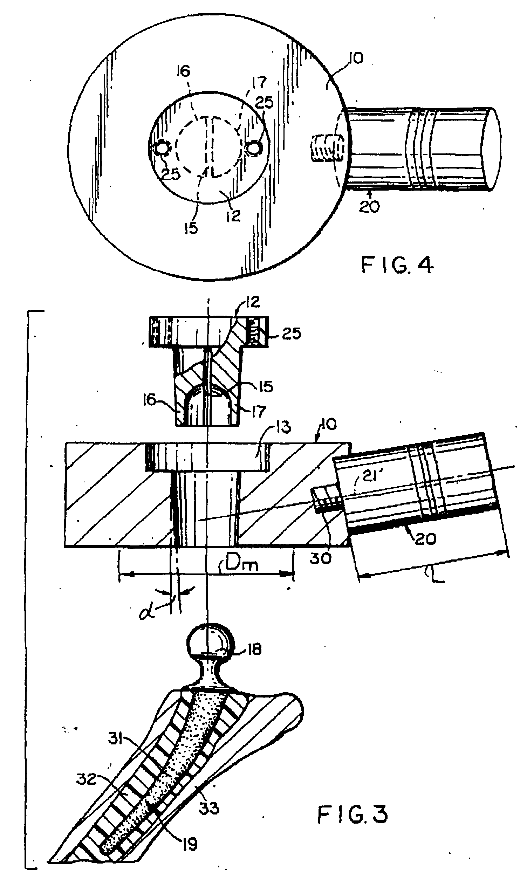

**Figs. 3 and 4** depict in greater detail an alternative version of the modification of **Fig. 2**, wherein the axial direction 21' of ultrasonic excitation from transducer 20 into body 10 is again radially inward and also downwardly tilted for anticipated near-center delivery to the spherical center of a chucked ball head 18. **In Fig. 3**, body 10 is again cylindrically annular as in **Fig. 1**, and the driving end of transducer 20 is received in a shallow, suitably inclined local bore in the periphery of body 10, for flat-to-flat end-face delivery of mechanical oscillation to body 10; in **Fig. 3**, a reduced stud portion 30 of the driving and face of the transducer is shown in tightly threaded engagement with a tapped bore at the base of the transducer-seating bore in body 10.

The greater detail of **Fig. 3** enables identification of further features common to all embodiments of the present invention. The threaded mounting via a stud is at 30, with otherwise flat-to-flat end interface from the transducer to the radial-mode body; such a flat-to-flat interface can be taken as presently preferred for all embodiments. The length L of the transducer should be an integer number of half-wavelengths of sound transmission in the medium of the transducer; this medium is preferably a conventional sandwich of aluminium alloy and stainless steel plate elements except of course for the piezo-electric ceramic disc and its wafer-thin electrodes which are at an outward offset from a central transverse plane of the transducer. The mean diameter Dₘ of the radial-mode body 10 is preferably such as to account for a mean geometrically circumferential extent (i.e. at diameter Dₘ) which is approximately an integer multiple of said wavelength. And the slightly convergent taper angle ∝ within the bore of body 10 is preferably in the range 1° to 2° . Anticipating substantial spherical concave-to-convex surface light engagement of the chuck or collet fingers 16, 17 to the ball or ball head 18, the concave spherical surfaces of fingers 16, 17 are preferably generated when fingers 16, 17 are radially inwardly displaced to the extent of an ultimately chucked state; and the outer-surface contouring of the chuck fingers 16, 17 is such as to develop progressive inwardly cantilevered bending to lock onto a ball or ball head 18 in the course of hammering or other axially jacked displacement into the fully chucked position shown for all embodiments except for the exploded diagram of **Fig. 3**.

Finally, **Fig. 3** illustrates the environment for use of a radial-mode system for all embodiments of the invention, namely, that the tapered stem portion 31 of the involved prosthetic device 19 has the environment of plastic cement 32, securing the prosthetic to and with an intramedullary cavity in a suitably cored and otherwise prepared proximal end of a femur 33. The relatively massive use of plastic cement 32 shown in **Fig. 3** will be understood to have been exaggerated, and it will be understood that very often in the preparation of a femur to receive a hip-joint prosthetic 19, the stem 31 will have been installed at least in part in such direct adjacency to bone tissue as to have involved bony-ingrowth into the prosthetic.

In the case of uncemented arthroplasty, the prosthesis will be attached by bone ingrowth and such attachment will be loosened or broken by relative vibrational movement at the bone/metal interface.

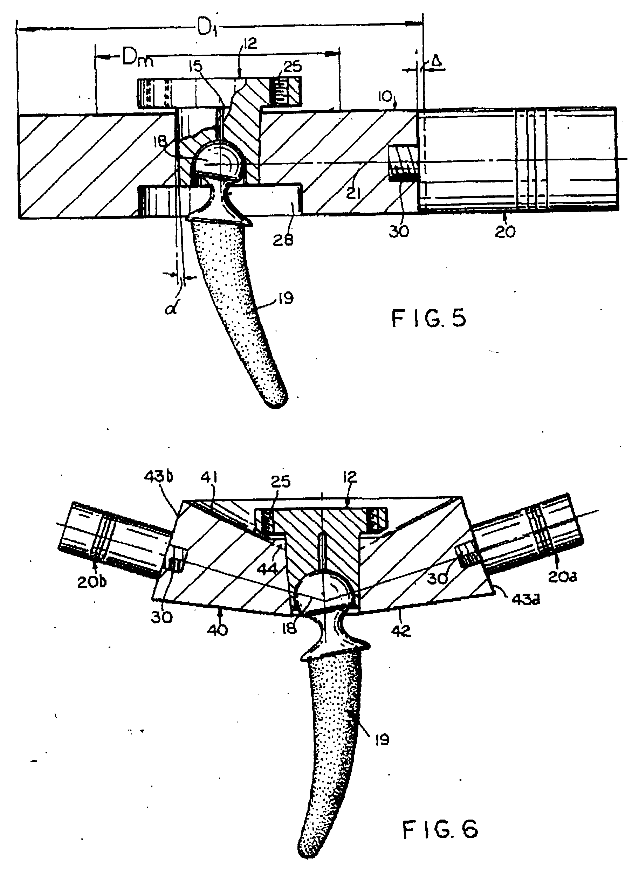

In the embodiment of **Fig. 5**, the radial-mode body 10 is right-cylindrical and of axial thickness matching the diameter of the transducer 20. There is no counterbore into which the flanged head of chuck 12 may be accommodated (as at 13 in **Figs. 1, 2 and 3**), but there is a lower counterbore 28 which enables clearance for chucked clamping of a prosthetic ball or ball head 10 with its spherical center substantially on the strictly radial transducer axis 21 of ultrasonic mechanical oscillation. Dimensioning applied to **Fig. 5** identifies the cylindrical outer diameter D of body 10 and the mean diameter Dₘ. further dimensioning at Δ identifies the fact that the body cylinder of diameter D is locally milled to a chordal flat (of radial depth Δ) for establishing a flat interface between transducer 20 and body 10, the same being tightly secured by threaded means 30.

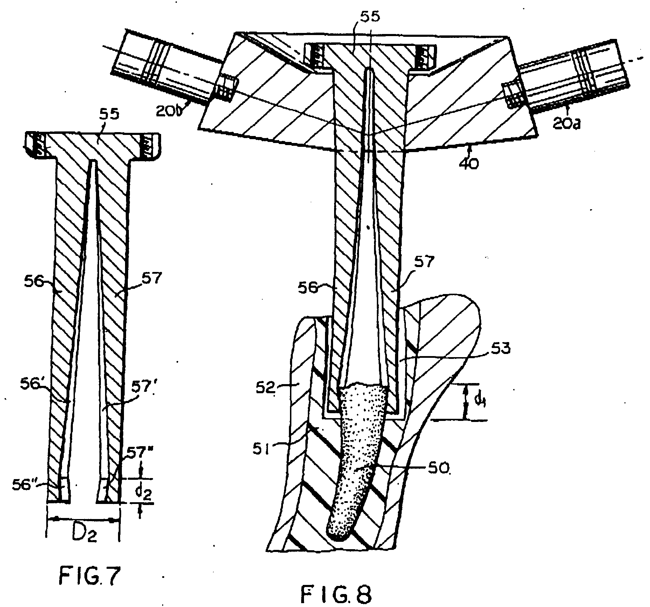

The embodiment of **Fig. 6** illustrates that a radial-mode annular body 40 of the invention need not be geometrically cylindrical, and it also illustrates that plural ultrasonic transducers may be provided at angular spacing around body 40, all with their respective axes of mechanical oscillation directed to substantially the centre of a ball or ball head 18 of an implanted prosthetic 19. As shown, the plurality of transducers is two, at 180° spacing about the central axis of body 40. The geometry of body 40 may be described as annular, with a central bore and chucking or collet means 12 as previously described. The annular body 40 features an upper concave frusto-conical end face 41 that is axially spaced from a lower convex frusto-conical end face 42. The outer surface of body 40 is also frustoconical except for local chordal flats 43a, 43b to accept the flat-interface relation of transducers 20a, 20b at their respective connections to body 40. Preferably, the concave slope of upper surface 41 is at greater offset from a radial plane with respect to the central axis of body 40 than is the lesser such offset in the case of a lower surface 42. This relationship establishes the presently preferred shape of body 40 as a dish wherein axial thickness reduces in approach to the central bore to which the chuck 12 is fitted, thus enabling radial-mode oscillation to bring resonant energy to even greater convergence at the desired locus of energy transfer to prosthetic 19. For purposes of deriving purely axial jacking force to dislodge a clamped condition of chuck 12, a small local fillet 44, one for each of the threaded jack bores 25 of the chuck flange, enables inserted jack bolts to be driven perpendicular to corresponding fillets 44.

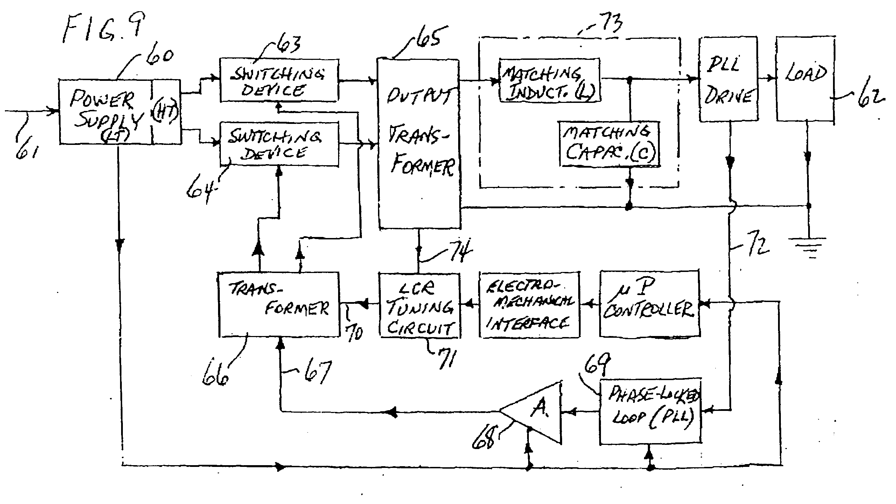

In the removal or attempted removal of a hip-joint or other prosthetic from a patient, it sometimes happens that the stem of the prosthetic breaks or is found to have been broken, thus leaving a distally embedded fragment of the stem, as shown at 50 in **Fig. 8**. At the point in time illustrated by **Fig. 8**, it will be understood that bone cement within the proximal end of a femur 52 has been selectively removed to establish an enlarged opening 53, i.e., enlarged from the socket of bone cement left upon removal of the proximal part of the prosthetic, and it will be further understood that this enlargement has been achieved not only as far as the embedded broken piece 50 but also to the extend d₁ therebeyond. Bone-cement removal tooling as described in **US Patent No. 5,536,266** is ideal for rapid and effective bone-cement removal but not to achieve enlargement, including to the predetermined depth d₁ beyond the location of the break which produced the embedded fragment 50.

Using a tool as described in the european Patent applications EP-A-617935 or EP-A-830854 to prepare an enlargement 53 in bone cement at the proximal end of femur 52, and the depth d₁ beyond the break responsible for the embedded fragment 50, all is then in readiness for use of a special tool bit as shown in **Fig. 7**, as a replacement for the chuck or collet element 12 in any of the embodiments described in connection with **Figs. 1, 2, 3, 5, and 6** above, and in **Fig. 8**, the tool bit of **Fig. 7** will be recognised in substitution for the chuck 12 of **Fig. 6**.

Briefly, the tool bit of **Fig. 7** is suitably of stainless steel and comprises a flanged head 55, integrally formed with plural elongate, relatively massive but tweezer like, legs 56, 57. The outer diameter D₂ may be cylindrical and thus constant in manufacture of the tool bit. Within this cylindrical outer-surface profile, a gradually tapering bore establishes concave inner-surfaces 56', 57' of the legs 56, 57, all except for the distal remainder d₂ which is characterised by short distally convergent concave profiles 56'', 57'' within the distal ends of legs 56, 57. The cylindrical diameter D₂ is selected to permit insertional entry of the distal ends of legs 56, 57 through the upper end of the convergent central bore of the radial-mode body, the same to be inwardly cammed in the course of full insertion through this central bore. This inward camming action deflects legs 56, 57 toward each other and in all likelihood into radially loaded mutual contact of their distal ends by the time these distal ends have been extended substantially fully beyond passage through the bore of body 40. At this point it is a simple matter to compliantly spread apart the distal ends of legs 56, 57 as by inserting and twisting a screwdriver blade there between, the thus achieved spread being such as to permit insertion of the distal ends of legs 56, 57 into the enlarged opening 53 and past the upper end of the embedded broken fragment 50. Such insertion may be to the extent d₂ beyond the detection of initial contact with fragment 50, whereupon the screwdriver or other spreading device may be removed to permit legs 56, 57 to apply a compliantly stressed grasp of the proximal end of fragment 50. At this point, the grip of legs 56, 57 on the fragment 50 is sufficient for direct transfer of ultrasonic energy from the radial-mode body 40, and via the tool bit of **Fig. 7**, to the fragment 50, whereby to impart ultrasonic energy to the fragment for melting or breaking its interface with bone cement or with bony ingrowth, as the case may be. And of course, when thus melted or broken at this interface, the embedded fragment 50 is in readiness for immediate extraction, using the same grasp by legs 56, 57.

The various embodiments of the invention will be seen to provide an apparatus for the removal of osteal prostheses, when exposed or broken as a fragment that may remain buried and embedded within bone. Importantly, the apparatus is served by a high-mass radial-mode resonator compressionally coupled to the spherical head, or cylindrical or Morse-tapered proximal end, of the prosthesis. The system meets two principal criteria: the radial-mode resonator permits axial attachment of a rigid mass to the prosthesis, without the trauma-inducing prospect of direct ultrasonic drive of the prosthetic device distally with respect to the central axis of the ball or ball-head and its embedded stem. This result is achieved with a relatively small shift in resonant frequency, and it is compatible with the use of a fully automatic tuning system such as that which is schematically presented in **Fig. 9**.

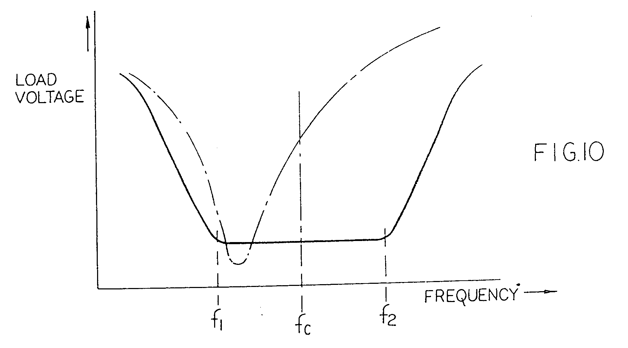

**Fig. 9** depicts excitation circuitry wherein phase-locked control expands the bandwidth tolerance of the system to shifts in mechanical resonance frequency, within limits, for a given setting of circuitry parameters. Specifically, a power-supply unit 60 may rely upon conventional a-c power, available at 61 as from a household wall outlet, and unit 60 derives both high-tension (HT) d-c and low-tension (LT) d-c supplies to automatic-tuning circuitry, wherein ultrasonic excitation voltage is delivered to the involved transducer 20, here symbolized as "load" 62. The HT supply is interrupted by phased high-frequency switching devices 63, 64, producing a square wave across the primary of an output transformer 66, the frequency of which is determined via a first input 67 suitably amplified at 68 from a phase-locked loop (PLL) at 69, and via a second input 70 from a microprocessor (µP) controlled LCR network 71, the LCR tuning circuit 71 is associated (via connection 74) with a low-voltage secondary winding of output transformer 65, and the tuning function of the microprocessor (µP) may be defined in software contained in an EEPROM or similar device, allowing automatic tuning over a wide frequency range. In the supply from transformer 65 to the load 63, matching values of inductance (L) and capacitance (C) are chosen to ensure sinusoidal current and voltage waveforms in the load 62 and to provide an essentially constant load-current characteristic. Feedback in line 72 reflects instantaneous output from the impedance-matching network 73 and is continuously supplied to the phase-locked loop means 69. The net result is indicated by the solid-line curve of **Fig. 10**, wherein phase-locked automatic tuning is seen to be ensured for the resonant-frequency band from f₁ to f₂ , indicating a span of mechanical resonance that is held in tune without need for manual adjustment; for comparison, the same structure and excitation values, without the benefit of the indicated phase-locked loop and automatic tuning are to be understood as producing the essentially single-tuned frequency characteristic that is shown by the phantom-line curve of **Fig. 10**.

## Claims

1. A radial-mode resonant device characterised in that it comprises an annular body (10,40) having a central axial bore (11) and an ultrasonic driver (20) having a central axis (21) of mechanically resonant oscillation directed generally radially inwardly with respect to the central axis of said body (10).

2. An ultrasonically driven tool comprising a collet means (12) in combination with a radial-mode resonant device as claimed in claim 1, characterised in that the collet means (12) comprises resiliently deformable means to define an engagement zone whereby energy from said resonant device may be delivered to a prosthesis.

3. An ultrasonically driven tool for use in revision arthroplasty as claimed in claim 2, for removing a prosthetic member at least partially encumbered by bone cement, characterised in that said collet means (12) is so adapted selectively to engage an exposed portion of said prosthetic member (19) with said body (10,40), and in that ultrasonic vibration with said oscillation axis (21) directed generally radially inwardly may be transmitted to said prosthetic member.

4. A tool as claimed in either claim 2 or claim 3, characterised in that said body (10,40) is a cylindrical annulus.

5. A tool as claimed any one of claims 2 to 4, characterised in that said body (10,40) has a mean diameter, intermediate an inner diameter and an outer diameter, where said mean diameter has a circular circumferential extent which is substantially an integral multiple of wavelengths at the frequency of mechanically resonant oscillation of said at least one driver.

6. A tool as claimed in any one of claims 2 to 5, characterised in that said body (40) is a dished solid having a concave conical first end face at one end of said bore (11) and a convex conical second end face at the opposite end of said bore, the connection of said at least one driver to said body (10) being such that the oscillation axis (21) is on such an alignment that, in addition to said generally radially inwardly direction, it is sloped in general to accord with the respective conical slopes of said end faces.

7. A tool as claimed in claim 6, characterised in that said first end face slopes with a conical apex angle that is less than that of said second end face, and in that the oscillation axis (21) is directed inwardly at a slope that is intermediate the slopes of said end faces.

8. A tool as claimed in any one of claims 2 to 7, characterised in that said collet (12) is provided with at least two longitudinally extending finger means (16,17) connected one to another at one longitudinal end, and arranged to engage at an opposite longitudinal end a portion of said prosthesis, and in that coacting cam or other formations on outer zones of said fingers (16,17) or on the surface of said bore (11) are arranged to cause engagement and releasable clamping of a portion of said prosthesis member.

9. A tool as claimed in claim 8, characterised in that said body (10) has a generally cylindrical bore (11), and said coacting cam formation may comprise a frusto-conical taper of said bore, and/or a radially outwardly directed flange at said one longitudinal end of said collet (12), or screw means acting on said flange.

## Patentansprüche

1. Radialmodus-Resonanzvorrichtung, dadurch gekennzeichnet, daß sie einen ringförmigen Körper (10,40) mit einer zentralen Axialbohrung (11) sowie einen Ultraschalltreiber (20) mit einer Zentralachse (21) und mit mechanischer Resonanzschwingung aufweist, die im wesentlichen radial nach innen in Bezug auf die Zentralachse des Körpers (10) gerichtet ist.

2. Ultraschallgetriebenes Werkzeug mit einer Spannhülseneinrichtung (12) in Kombination mit einer Radialmodus-Resonanzvorrichtung nach Anspruch 1, dadurch gekennzeichnet, daß die Spannhülsenvorrichtung (12) eine elastisch verformbare Einrichtung zum Festlegen einer Eingriffszone aufweist, durch die Energie von der Resonanzvorrichtung an eine Prothese abgegeben werden kann.

3. Ultraschallgetriebenes Werkzeug zur Anwendung bei Revisions-Arthroplastik gemäß Anspruch 2 zum Entfernen eines Prothesenelements, das zumindest teilweise von Knochenzement gefüllt ist, dadurch gekennzeichnet, daß die Spanneinrichtung (12) für selektive Verbindung eines freiliegenden Anschnitts des Prothesenelements (19) mit dem Körper (10,40) geeignet ist, und daß bei im wesentlichen radial nach innen gerichteter Schwingungsachse (21) Ultraschallschwingung auf das Prothesenelement übertragen werden kann.

4. Werkzeug gemäß Anspruch 2 oder 3, dadurch gekennzeichnet, daß der Körper (10,40) ein zylindrischer Ring ist.

5. Werkzeug gemäß einem der Ansprüche 2 bis 4, dadurch gekennzeichnet, daß der Körper (10,40) einen mittleren Durchmesser zwischen einem Innendurchmesser und einem Außendurchmesser hat, wobei der mittlere Durchmesser eine Kreisumfangserstreckung aufweist, die im wesentlichen ein ganzzahliges Vielfaches der Wellenlängen der Frequenz der mechanischen Resonanzschwingung des mindestens einen Treibers ist.

6. Werkzeug gemäß einem der Ansprüche 2 bis 5, dadurch gekennzeichnet, daß der Körper (40) ein teller- bzw. schüsselförmiger Festkörper mit einer konkaven, konischen ersten Endfläche am einen Ende der Bohrung (11) und einer konvexen, konischen zweiten Endfläche am entgegengesetzten Ende der Bohrung ist, wobei die Verbindung des mindestens einen Treibers mit dem Körper (10) so ausgelegt ist, daß die Schwingungsachse (21) derart ausgerichtet ist, daß sie zusätzlich zu der im wesentlichen radial nach innen verlaufenden Richtung allgemein bzw. insgesamt geneigt ist, um mit den betreffenden konischen Neigungen der Endflächen übereinzustimmen.

7. Werkzeug gemäß Anspruch 6, dadurch gekennzeichnet, daß die erste Endfläche mit einem konischen Scheitelpunktwinkel geneigt ist, der geringer ist als der der zweiten Endfläche, und daß die Schwingungsachse (21) mit einer Neigung nach innen gerichtet ist, die zwischen den Neigungen der Endflächen liegt.

8. Werkzeug gemäß einem der Ansprüche 2 bis 7, dadurch gekennzeichnet, daß die Spannhülseneinrichtung (12) mit mindestens zwei sich in Längsrichtung erstreckenden Fingermitteln (16,17) versehen ist, die miteinander an einem Längsende verbunden sind, und zum Angriff mit einem entgegengesetzten Längsende an einem Teil der Prothese angeordnet ist, und daß zusammenwirkende Nocken- oder andere Formationen an Außenzonen der Finger (16,17) oder an der Oberfläche der Bohrung (11) zum Bewirken eines Angriffs und lösbaren Festhaltens eines Teils des Prothesenelements angeordnet sind.

9. Werkzeug gemäß Anspruch 8, dadurch gekennzeichnet, daß der Körper (10) eine im wesentlichen zylindrische Bohrung (11) aufweist, und die zusammenwirkenden Nockenformationen eine kegelstumpfförmige Verjüngung/Ausweitung der Bohrung und/oder einen radial nach außen gerichteten Flansch an dem einen Längsende der Spannhülseneinrichtung (12) oder ein auf den Flansch einwirkendes Schraubenmittel umfassen können0.

## Revendications

1. Dispositif de résonance à mode radial caractérisé en ce qu'il comprend un corps annulaire (10, 40) ayant un alésage axial central (11) et un dispositif d'excitation ultrasonique (20) ayant un axe central (21) d'oscillation de résonance mécanique dirigé en général radialement vers l'intérieur par rapport à l'axe central dudit corps (10).

2. Outil à ultrasons comprenant un moyen de serrage (12) en combinaison avec un dispositif de résonance à mode radial selon la revendication 1, caractérisé en ce que le moyen de serrage (12) comprend un moyen déformable de façon élastique pour définir une zone d'enclenchement par laquelle l'énergie dudit dispositif de résonance peut être délivrée à une prothèse.

3. Outil à ultrasons à utiliser lors d'une arthroplastie de révision selon la revendication 2, pour le retrait d'un élément prothétique au moins partiellement encombré de ciment chirurgical, caractérisé en ce que ledit moyen de serrage (12) est conçu de façon sélective de manière à enclencher une partie à nu dudit élément prothétique (19) avec ledit corps (10, 40) et en ce que la vibration ultrasonique avec ledit axe d'oscillation (21) dirigé en général radialement vers l'intérieur peut être transmise audit élément prothétique.

4. Outil selon la revendication 2 ou la revendication 3, caractérisé en ce que ledit corps (10, 40) est un anneau cylindrique.

5. Outil selon l'une quelconque des revendications 2 à 4, caractérisé en ce que ledit corps (10, 40) est doté d'un diamètre moyen, entre un diamètre intérieur et un diamètre extérieur, où ledit diamètre moyen a une étendue en circonférence circulaire qui est substantiellement un multiple entier des longueurs d'onde à la fréquence d'oscillation de résonance mécanique d'au moins un circuit d'excitation.

6. Outil selon l'une quelconque des revendications 2 à 5, caractérisé en ce que ledit corps (40) est un solide embouti ayant une première surface d'extrémité conique concave à une extrémité dudit alésage (11) et une deuxième surface d'extrémité conique convexe à l'extrémité opposée dudit alésage, la liaison dudit au moins premier circuit d'excitation audit corps (10) étant telle que l'axe d'oscillation (21) est dans un alignement tel qu'en plus de ladite direction en général radiale vers l'intérieur, il est incliné pour en général s'accorder avec les pentes coniques respectives desdites surfaces d'extrémité.

7. Outil selon la revendication 6, caractérisé en ce que ladite première surface d'extrémité est inclinée avec un angle d'apex de conicité qui est inférieur à celui de ladite deuxième surface d'extrémité, et en ce que l'axe d'oscillation (21) est dirigé vers l'intérieur avec une pente qui se situe entre les pentes des deux surfaces d'extrémité.

8. Outil selon l'une quelconque des revendications 2 à 7, caractérisé en ce que ledit serrage (12) est muni d'au moins deux moyens formant des doigts s'étendant longitudinalement (16, 17) reliés l'un à l'autre à une extrémité longitudinale, et faits pour s'enclencher sur une partie d'extrémité longitudinale opposée de ladite prothèse, et en ce qu'une came ou autres structures agissant en coopération avec les zones extérieures desdits doigts (16, 17) ou sur la surface dudit alésage (11) sont faites pour favoriser l'enclenchement et le bridage déblocable d'une portion dudit élément de prothèse.

9. Outil selon la revendication 8, caractérisé en ce que ledit corps (10) est doté d'un alésage en général cylindrique (11), et ladite structure de came agissant avec peut comprendre un rétrécissement tronconique dudit orifice, et/ou un flasque dirigé radialement vers l'extérieur au niveau d'une des extrémités longitudinales dudit moyen de serrage (12) ou à vis agissant sur ledit flasque.
